# EUROPEAN PATENT APPLICATION

(11) **EP 1 405 851 A1**
(43) Date of publication of application: **07.04.2004**
(21) Application number: 02292421.1
(22) Date of filing: 02.10.2002
(51) Int. Cl.: C07D 321/10, C07D 319/20

(54) **Method for producing seven-membered diether compounds and intermediates thereof**

(71) Applicant: Takasago International Corporation, Tokyo-to 144-8721 (JP)
(72) Inventor: Susarte Rogel, Manuel, El Palmar, 30120 Murcia (ES); De Mora Navarro, Javier, El Palmar, 30120 Murcia (ES); Alajarin Ceron, Mateo, Organic dev.,Faculty of, 30100 Murcia (ES); Akira, Amano, c/oTakasago Intern.Corp., Hiratsuka-shi, Kanagawa-ken 254-0073 (JP)
(74) Representative: Uchida, Kenji

(57) **Abstract**

The invention provides a method for preparing 3,4-dihydro(2H)-1,5-benzodioxepin-3-ones represented by the formula (3): 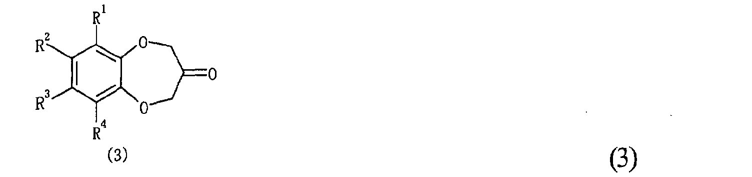 wherein R¹, R², R³ and R⁴ respectively represent, independently, a hydrogen atom an alkyl group or an alkenyl group, and R¹, R², R³ and R⁴ as a whole do not comprise more than 10 carbon atoms and that, when the two adjacent groups respectively comprise an alkyl group or an alkenyl group, they may be combined together to form a carbon ring; by reacting either a compound represented by the formula (1a) and/or (1b): 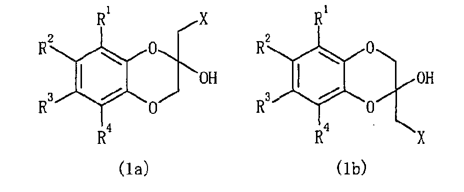 wherein X represent a halogen atom; and R¹, R², R³ and R⁴ are as defined above; or catechols represented by the formula (5): 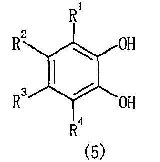 wherein R¹, R², R³ and R⁴ are as defined above;
with 1,3-dihaloacetones in the presence of sodium carbonate, through the removal of hydrogen halide.

## Description

The present invention relates to a method for producing 3,4-dihydro(2H)-1,5-benzodioxepin-3-ones which are useful as synthetic fragrances, medicines or the like. The invention further concerns a method for preparing intermediate products for preparing the above end products.

A large number of natural fragrance sources such as flowers, fruits, musk or the like have been used since early times for savoring aroma, or for medicinal or religious purposes.

With the development of synthetic chemistry in recent years, a large number of synthetic fragrance materials have been produced and used as useful fragrance sources. Such synthetic fragrances are described e.g. by Steffen Archtander in "Perfume and Flavor Chemistry", vol.2, 1969.

Recently, the olfactory sense for aromas has increasingly diversified. To satisfy this tendency, research into fragrances with further new aromas has become a preferred subject for researches. Amongst these fragrances, a so-called marine note or ozone note fragrance is particularly appreciated for its aromatic quality which recalls the image of the sea.

A key component for such fragrances with a marine note was reported as 7-methyl-3,4-dihydro(2H)-1, 5-benzodioxepin-3-one in U.S. Patent 3,517,031, and this product has since been used widely in various cosmetics.

Methods of synthesizing 3,4-dihydro(2H)-1,5-benzodioxepin-3-one have also been reported. According to a first method, catechol is first allowed to react in the co-presence of 1,3-dichloro-2-propanol and potassium hydroxide, thereby producing 3,4-dihydro(2H)-1,5-benzodioxepin-3-ol. The latter compound is then oxidized. However, the overall yield of the product according to this method is a meager 4% (cf. *Tetrahedron,* 1962 (18), 289). In this document, it is further described that the reaction of catechol with 1,3-dichloroacetone does not produce the sought-after product.

According to a second method reported in U.S. Patent 3,517,031, 7-methyl-3,4-dihydro(2H)-1,5-benzodioxepin-3-one (10) is synthesized by first preparing a diacetic acid ester (8) through the reaction of 4-methylcatechol (7) with methyl chloroacetate or methyl bromoacetate, then performing a Dickmann reaction in the presence of sodium hydride or another strongly basic reagent, thereby converting the ester (8) into a 2-carbomethoxy compound (9), and finally heating the compound (9) in ethanol with hydrochloric acid.

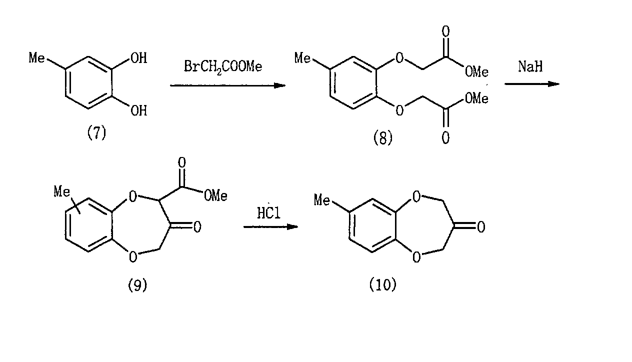

However, the above method has drawbacks when applied in the production line, since sodium hydride or the like, used in the method, requires severe reaction conditions and careful handling. Further, this method gives only a very low yield.

A third method for synthesizing 3,4-dihydro(2H)-1,5-benzodioxepin-3-one, via the reactions shown in scheme below, is described in *Canadian* Journal *of the Chemistry,* vol.53, no.15, pp.2279-93 (1975).

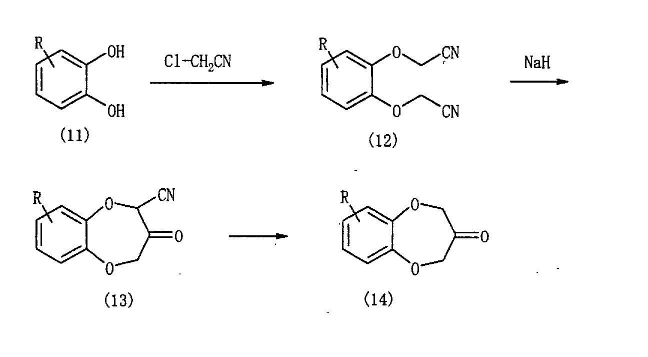

However, this method has the same disadvantages as the preceding method, in that the same kinds of reagent and reaction condition as the previous ones are used. The product yield is also low. Consequently, this method is not adapted to an industrial production method either.

An object of the present invention is therefore to provide a method which can solve the aforementioned problems. According to the present invention, 3,4-dihydro(2H)-1,5-benzodioxepin-3-one can be produced under mild reaction conditions, with high yield and at low production costs.

According to the invention, catechol represented by the formula (5) is reacted with 1,3-dihaloketones represented by the formula (6) in the presence of sodium carbonate, so that compound(s) represented by the formula (1a) and/or formula (1b) is/are formed. Subsequently, the compound(s) (1a) and/or (1b) is/are efficiently converted into 3,4-dihydro(2H)-1,5-benzodioxepin-3-ones represented by the formula (3) in the presence of sodium carbonate. Furthermore, according to the above method, 3,4-dihydro(2H)-1,5-benzodioxepin-3-ones can be obtained in one reaction step. The compounds (1a) and (1b) are new compounds that enable the above one-step synthesis method.

To the above end, there is provided a method for producing 3,4-dihydro(2H)-1,5-benzodioxepin-3-one represented by the formula (3): 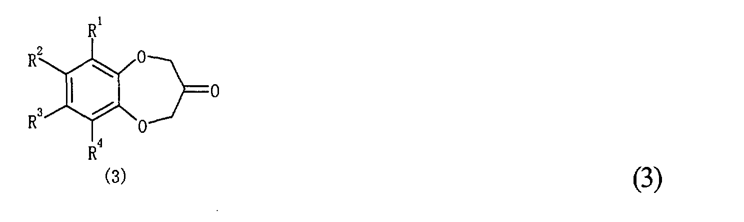 wherein R¹, R², R³ and R⁴ respectively represent, independently, a hydrogen atom, an alkyl group or an alkenyl group, with the proviso that R¹, R², R³ and R⁴ as a whole do not comprise more than 10 carbon atoms and that, when the two adjacent groups respectively comprise an alkyl group or an alkenyl group, they may be combined together to form a carbon ring;
the method comprising the step of reacting catechols represented by the formula (5): 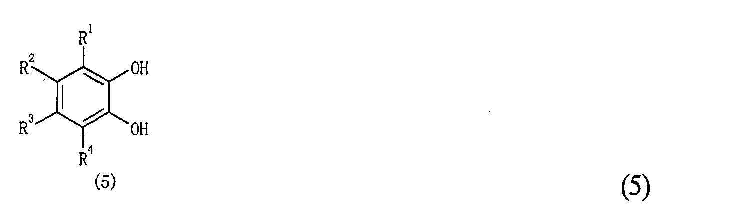 wherein R¹, R², R³ and R⁴ are as defined above;
with 1,3-dihaloacetones represented by the formula (6): 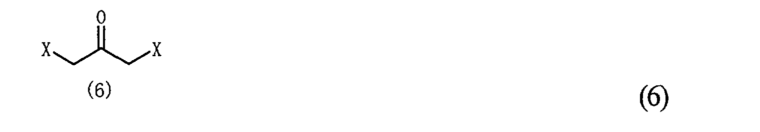 wherein the two letters X represent, independently, a halogen atom;
in the presence of sodium carbonate, through the removal of hydrogen halide.

The invention also relates to a method for producing 2-hydroxy-2-halogenomethyl-1,4-benzodioxane derivatives represented by the formula (1a): 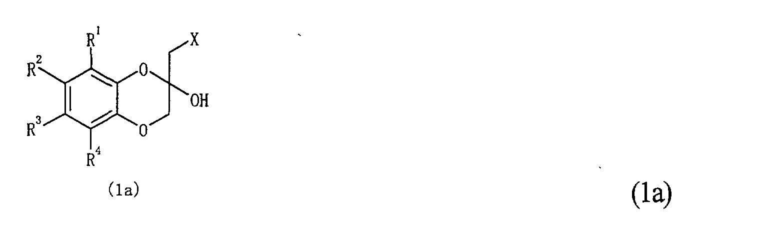 wherein X represents a halogen atom, and R¹, R², R³ and R⁴ respectively represent, independently, a hydrogen atom, an alkyl group or an alkenyl group, with the proviso that R¹, R², R³ and R⁴ as a whole do not comprise more than 10 carbon atoms and that, when the two adjacent groups respectively comprise an alkyl group or an alkenyl group, they may be combined together to form a carbon ring;
and/or 2-hydroxy-2-halogenomethyl-1,4-benzodioxane derivatives represented by the formula (1b): 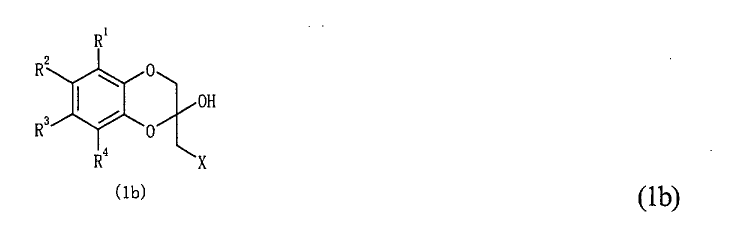 wherein R¹, R², R³, R⁴ and X are as defined above;
the method comprising the step of reacting catechols represented by the formula (5): 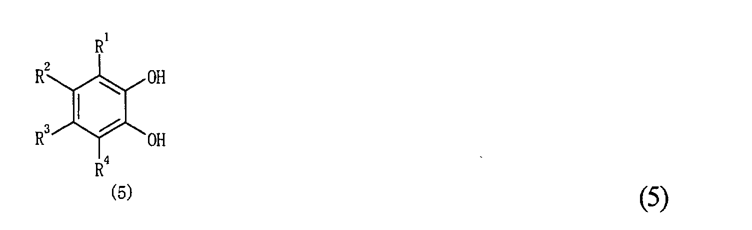 wherein R¹, R², R³ and R⁴ are as defined above;
with 1,3-dihaloacetones represented by the formula (6): 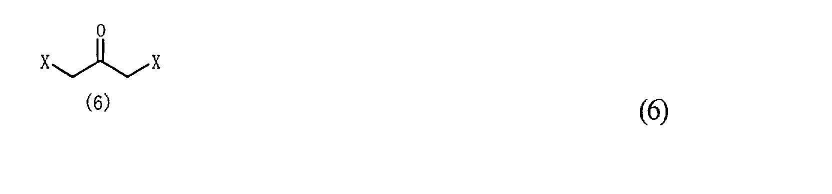 wherein the two letters X represent, independently, a halogen atom;
in the presence of sodium carbonate, through the removal of hydrogen halide.

In the above methods, the reacting step preferably comprises reacting the catechols with 1,3-dihaloacetones in which the halogen atom comprises a chlorine atom or a bromine atom..

Further, the reacting step preferably comprises reacting, with the 1,3-dihaloacetones, catechols in which R¹ and R⁴ are hydrogen atoms and either R² or R³ is a hydrogen atom and the other is an alkyl group or alkenyl group.

The invention further concerns a method for producing 3,4-dihydro(2H)-1,5-benzodioxepin-3-one represented by the formula (3): 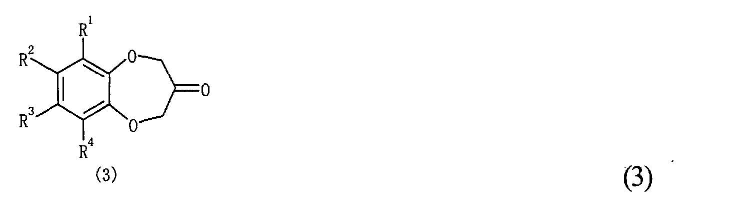 wherein R¹, R², R³ and R⁴ respectively represent, independently, a hydrogen atom, an alkyl group or an alkenyl group, with the proviso that R¹, R², R³ and R⁴ as a whole do not comprise more than 10 carbon atoms and that, when the two adjacent groups respectively comprise an alkyl group or an alkenyl group, they may be combined together to form a carbon ring;
the method comprising the step of reacting at least one compound represented by the formula (1a): 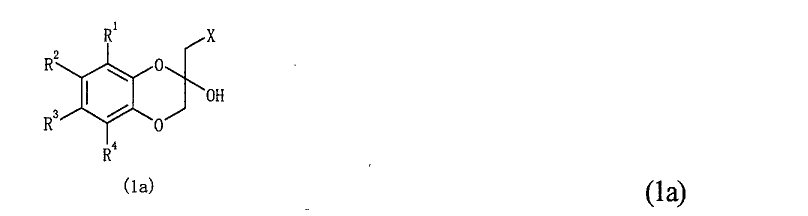 wherein X represents a halogen atom; and R¹, R², R³ and R⁴ are as defined above;
or at least one compound represented by the formula (1b): 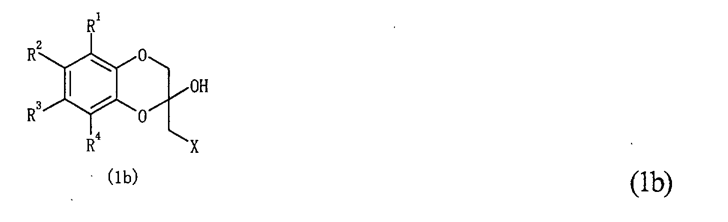 wherein X, R¹, R², R³ and R⁴ are as defined above;
or a mixture thereof;
with sodium carbonate, through the removal of hydrogen halide.

Preferably, the reacting step comprises reacting, with the sodium carbonate, at least one compound of formula (1a) or (1b) in which the halogen atom comprises a chlorine atom or a bromine atom.

In the above methods, the reacting step preferably comprises reacting, with the sodium carbonate, at least one compound of formula (1a) or (1b) in which R¹ and R⁴ are hydrogen atoms and either R² or R³ is a hydrogen atom and the other is an alkyl group or alkenyl group.

There is further provided a 2-hydroxy-2-halogenomethyl-1,4-benzodioxane derivative represented by the formula (1a): 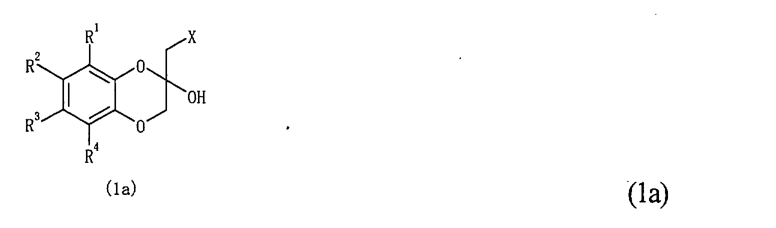 wherein X represents a halogen atom; R¹, R², R³ and R⁴ respectively represent, independently, a hydrogen atom, an alkyl group or an alkenyl group, with the proviso that R¹, R², R³ and R⁴ as a whole do not comprise more than 10 carbon atoms and that, when the two adjacent groups respectively comprise an alkyl group or an alkenyl group, they may be combined together to form a carbon ring;
or represented by the formula (1b): 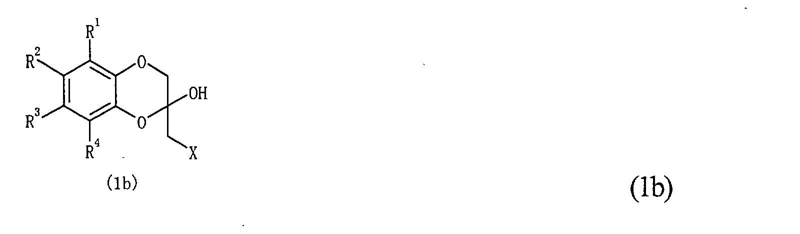 wherein X, R¹, R², R³ and R⁴ are as defined above.

In the above 2-hydroxy-2-halogenomethyl-1,4-benzodioxane derivative, the halogen atom preferably comprises a chlorine atom or a bromine atom.

Preferably yet, in the above derivative, R¹ and R⁴ are hydrogen atoms and either one of R² and R³ is hydrogen atom and the other is an alkyl group or alkenyl group.

The above, and the other objects, features and advantages of the present invention will be made apparent from the following description of the preferred embodiments, given as non-limiting examples.

A first aspect of the invention relates to the preparation of the compounds represented by the formula (1a): 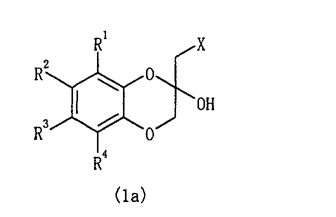 wherein X represents a halogen atom, e.g. a fluorine atom, a chlorine atom, a bromine atom, an iodine atom and the like, preferably a chlorine atom or a bromine atom; and R¹, R², R³ and R⁴ respectively represent, independently from one another, a hydrogen atom, an alkyl group or an alkenyl group, with the proviso that R¹, R², R³ and R⁴ as a whole do not comprise more than 10 carbon atoms and that, when the two adjacent groups respectively comprise an alkyl group or an alkenyl group, they may be combined together to form a carbon ring;
and the compounds represented by the formula (1b): 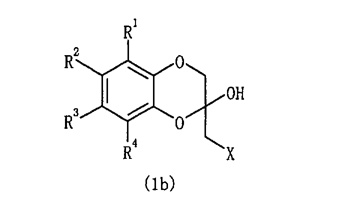 wherein X, R¹, R², R³ and R⁴ are as defined above.

The substituent groups on the benzene rings of 2-hydroxy-2-halogenomethyl-1,4-benzodioxane derivatives represented by the formula (1a) and formula (1b) are not particularly limited, insofar as they respectively represent, independently from one another, a hydrogen atom, an alkyl group or an alkenyl group, with the proviso that R¹, R², R³ and R⁴ as a whole do not comprise more than 10 carbon atoms, and are stable under the reaction conditions of the present invention.

The alkyl group may be linear or branched and include, for example, 1 to 6 carbon atoms. Specific examples of such an alkyl group include a methyl group, an ethyl group, a propyl group, an isopropyl group, a butyl group, a secondary butyl group (hereinafter s-butyl group), a tertiary butyl group (hereinafter t-butyl group), an isobutyl group, a pentyl group, a 2-methylpentyl group, a 3-methylpentyl group, a hexyl group and the like. The alkenyl group may be linear or branched and include, for example, 2 to 6 carbon atoms. Specific examples of the alkenyl group include a 2-propenyl group, a 2-butenyl group, a 3-hexenyl group and the like.

In cases where the two adjacent groups, i.e. R¹ and R², and/or R³ and R⁴, or R² and R³, respectively comprise, independently, an alkyl group or an alkenyl group, they may be combined together to form a carbon ring. For instance, R² may be combined with R³ to form a ring, e.g. a six-membered carbon ring.

Examples of such a ring include a cyclohexene ring, a benzene ring and the like.

The letters X in the formulae (1a), (1b) and (6) represent, independently, a halogen atom such as a fluorine atom, a chlorine atom, a bromine atom, an iodine atom and the like, preferably a chlorine atom or a bromine atom.

Specific examples of the compounds (1a) and (1b), where X is chlorine atom, include:
2-hydroxy-2-chloromethyl-1,4-benzodioxane;
2-hydroxy-2-chloromethyl-8-methyl-1,4-benzodioxane;
2-hydroxy-2-chloromethyl-7-methyl-1,4-benzodioxane;
2-hydroxy-2-chloromethyl-6-methyl-1,4-benzodioxane;
2-hydroxy-2-chloromethyl-5-methyl-1,4-benzodioxane;
2-hydroxy-2-chloromethyl-7,8-dimethyl-1,4-benzodioxane;
2-hydroxy-2-chloromethyl-7-ethyl-1,4-benzodioxane;
2-hydroxy-2-chloromethyl-6-(isopropyl)-1,4-benzodioxane;
2-hydroxy-2-chloromethyl-7-(isopropyl)-1,4-benzodioxane;
2-hydroxy-2-chloromethyl-6-propyl-1,4-benzodioxane;
2-hydroxy-2-chloromethyl-7-(2-propenyl)-1,4-benzodioxane;
2-hydroxy-2-chloromethyl-6-butyl-1,4-benzodioxane;
2-hydroxy-2-chloromethyl-7-butyl-1,4-benzodioxane;
2-hydroxy-2-chloromethyl-6-(s-butyl)-1,4-benzodioxane;
2-hydroxy-2-chloromethyl-7-(s-butyl)-1,4-benzodioxane;
2-hydroxy-2-chloromethyl-6-(t-butyl)-1,4-benzodioxane;
2-hydroxy-2-chloromethyl-7-(t-butyl)-1,4-benzodioxane;
2-hydroxy-2-chloromethyl-7-(2-methylbutyl)-1,4-benzodioxane;
2-hydroxy-2-chloromethyl-6-(3-methylbutyl)-1,4-benzodioxane;
2-hydroxy-2-chloromethyl-7-pentyl-1,4-benzodioxane;
2-hydroxy-2-chloromethyl-6-(2-methylpentyl)-1,4-benzodioxane;
2-hydroxy-2-chloromethyl-7-(3-methylpentyl)-1,4-benzodioxane;
2-hydroxy-2-chloromethyl-6-(4-methylpentyl)-1,4-benzodioxane;
2-hydroxy-2-chloromethyl-7-hexyl-1,4-benzodioxane; etc..

As to examples of the compounds (1a) and (1b), where X is bromine atom, chlorine in the above examples may be replaced by bromine.

Among the above compounds, preferred embodiments are those in which R¹ and R⁴ represent a hydrogen atom, and either R² or R³ is a hydrogen atom and the other is an alkyl group.

Examples of such preferable compounds include:
2-hydroxy-2-chloromethyl-7-methyl-1,4-benzodioxane;
2-hydroxy-2-bromomethyl-7-methyl-1,4-benzodioxane;
2-hydroxy-2-chloromethyl-6-methyl-1,4-benzodioxane;
2-hydroxy-2-bromomethyl-6-methyl-1,4-benzodioxane;
2-hydroxy-2-chloromethyl-7-ethyl-1,4-benzodioxane;
2-hydroxy-2-bromomethyl-7-(isopropyl)-1,4-benzodioxane;
2-hydroxy-2-chloromethyl-7-(isopropyl)-1,4-benzodioxane;
2-hydroxy-2-bromomethyl-7-ethyl-1,4-benzodioxane;
2-hydroxy-2-chloromethyl-6-propyl-1,4-benzodioxane;
2-hydroxy-2-bromomethyl-6-propyl-1,4-benzodioxane;
2-hydroxy-2-chloromethyl-6-butyl-1,4-benzodioxane;
2-hydroxy-2-bromomethyl-6-butyl-1,4-benzodioxane;
2-hydroxy-2-chloromethyl-7-(2-methylbutyl)-1,4-benzodioxane;
2-hydroxy-2-bromomethyl-7-(2-methylbutyl)-1,4-benzodioxane;
2-hydroxy-2-chloromethyl-6-(3-methylbutyl)-1,4-benzodioxane;
2-hydroxy-2-bromomethyl-6-(3-methylbutyl)-1,4-benzodioxane;
2-hydroxy-2-chloromethyl-7-pentyl-1,4-benzodioxane;
2-hydroxy-2-bromomethyl-7-pentyl-1,4-benzodioxane;
2-hydroxy-2-chloromethyl-6-(2-methylpentyl)-1,4-benzodioxane;
2-hydroxy-2-bromomethyl-6-(2-methylpentyl)-1,4-benzodioxane;
2-hydroxy-2-chloromethyl-7-(3-methylpentyl)-1,4-benzodioxane;
2-hydroxy-2-bromomethyl-7-(3-methylpentyl)-1,4-benzodioxane;
2-hydroxy-2-bromomethyl-6-(4-methylpentyl)-1,4-benzodioxane;
2-hydroxy-2-chloromethyl-7-hexyl-1,4-benzodioxane; and
2-hydroxy-2-bromomethyl-7-hexyl-1,4-benzodioxane.

Examples of more preferable compounds include:
2-hydroxy-2-chloromethyl-7-methyl-1,4-benzodioxane;
2-hydroxy-2-bromomethyl-7-methyl-1,4-benzodioxane;
2-hydroxy-2-chloromethyl-6-methyl-1,4-benzodioxane;
2-hydroxy-2-bromomethyl-6-methyl-1,4-benzodioxane; etc..

The compounds (1a) and (1b) can be easily converted into 3,4-dihydro(2H)-1,5-benzodioxepin-3-one derivatives in the presence of sodium carbonate, and thus constitute important intermediate products for their synthesis.

The compounds (1a) and/or (1b) are prepared, e.g., by reacting catechols of formula (5) with 1,3-dihaloacetones of formula (6), and eliminating one hydrogen halide molecule, as shown in Scheme 1 below.

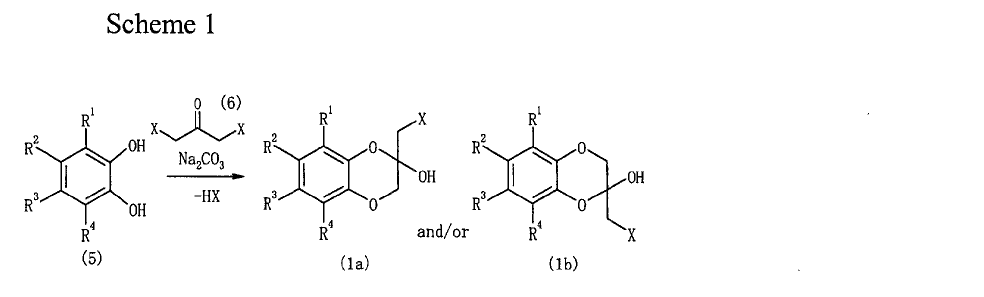

In the above reaction, the resulting 2-hydroxy-2-halomethyl-1,4-benzodioxanes are generally obtained as a mixture of compounds of formulae (1a) and (1b).

In particular, to obtain the compounds of formulae (1a) and (1b), catechols of formula (5) are reacted with 1,3-dihaloacetones of formula (6) in a solvent in the presence of sodium carbonate and, optionally, a reaction accelerating agent.

In the formula (5), each of R¹, R², R³ and R⁴ independently represents a hydrogen atom, an alkyl group or an alkenyl group, wherein, when the alkyl groups, the alkenyl groups or the alkyl group and alkenyl group are placed adjacent, they may be combined together to form a new carbon ring. However, the total number of carbon atoms contained in R¹, R², R³ and R⁴ does not exceed 10.

Examples of catechols represented by the formula (5) include catechol, 3-methylcatechol, 4-methylcatechol, 3,4-dimethylcatechol, 3,5-dimethylcatechol, 4-ethylcatechol, 4-propylcatechol, 4-isopropylcatechol, 4-(2-propenyl)catechol, 4-butylcatechol, 4-(2-methylbutyl)catechol, 4-(3-methylbutyl)catechol, 4-pentylcatechol, 4-(2-methylpentyl)catechol, 4-(3-methylpentyl)catechol, 4-(4-methylpentyl)catechol, 4-hexylcatechol, 2,3-dihydroxynaphthalene, 6,7-dihydroxy-1,2,3,4-tetrahydronaphthalene and the like.

In particular, compounds in which R¹ and R⁴ represent a hydrogen atom and either R² or R³ represents a hydrogen atom and the other represents an alkyl group or an alkenyl group, are preferably used.

Examples of such compounds include 4-methylcatechol, 4-ethylcatechol, 4-propylcatechol, 4-isopropylcatechol, 4-(2-propenyl)catechol, 4-butylcatechol, 4-(2-methylbutyl)catechol, 4-(3-methylbutyl)catechol, 4-pentylcatechol, 4-(2-methylpentyl)catechol, 4-(3-methylpentyl)catechol, 4-(4-methylpentyl)catechol, 4-hexylcatechol and the like can be cited.

Amongst them, the most preferred is 4-methylcatechol.

In 1,3-dihaloacetones of formula (6), each of the two letters X represents, independently, a halogen atom such as a fluorine atom, a chlorine atom, a bromine atom, an iodine atom and the like, preferably a chlorine atom or a bromine atom.

Examples of 1,3-dihaloacetones of formula (6) include dichloroacetone, dibromoacetone and 1-bromo-3-chloroacetone, preferably dichloroacetone or a dibromoacetone, but are not limited thereto.

Catechols of formula (5) and 1,3-dihaloacetones of formula (6) are added to the reaction in a molar ratio ranging from about 1 : 1 to about 1 : 1.5, preferably from about 1 : 1.1 to about 1 : 1.3.

Examples of sodium carbonate to be used in the reaction include anhydrous sodium carbonate and sodium carbonate monohydrate. They are preferably prepared into fine powder particles. Sodium carbonate is added in an amount ranging from about 1 to 5 equivalents, preferably from about 1.5 to 3 equivalents, relative to catechols of formula (5).

Examples of reaction accelerating agent include iodine salts e.g. sodium iodide and potassium iodide, phase-transfer catalysts, and the like. The reaction accelerator is preferably used in an amount ranging from about 0.01 to about 1.0 equivalent, preferably from about 0.05 to about 0.25 equivalent, relative to 1,3-dihaloacetones.

Solvents to be used in the reaction are not particularly limited, insofar as they do not affect the reaction. Examples of suitable solvents include water; alcohols such as methanol and ethanol; esters such as ethyl acetate; ketones such as acetone, methyl ethyl ketone, methyl isobutyl ketone and cyclohexanone; ethers such as diisopropyl ether, tetrahydrofuran and dioxane; aromatic hydrocarbons such as benzene, toluene and xylene; amides such as dimethylformamide; dimethyl sulfoxide; and the like. The solvents may be used alone or as a mixture thereof. Among the above-mentioned solvents, ketones e.g. acetone, methyl ethyl ketone, methyl isobutyl ketone and cyclohexanone are preferably used. The amount of solvent is not particularly limited. However, to facilitate industrial production, they are used in a weight ratio ranging from about 3 : 1 to about 100 : 1, preferably from about 5 : 1 to about 50 : 1, relative to catechols (5).

The reaction temperature is usually comprised between about 30°C and about 100°C, preferably between about 50°C and about 80°C. The length of reaction time is not particularly limited. However, to facilitate industrial production, it is usually selected between about 1 and about 15 hours, preferably between about 3 and about 10 hours.

The reaction can be carried out in air, but preferably in an inert gas atmosphere. Examples of inert gas include nitrogen gas, argon gas and the like.

A second aspect of the invention concerns converting 2-Hydroxy-2-halogenomethyl-1,4-benzodioxane derivatives of formula(e) (1a) and/or (1b) into 3,4-dihydro(2H)-1,5-benzodioxepin-3-one derivatives of formula (3) by reacting the former with sodium carbonate in a solvent, so as to remove hydrogen halide, as shown in Scheme 2 below.

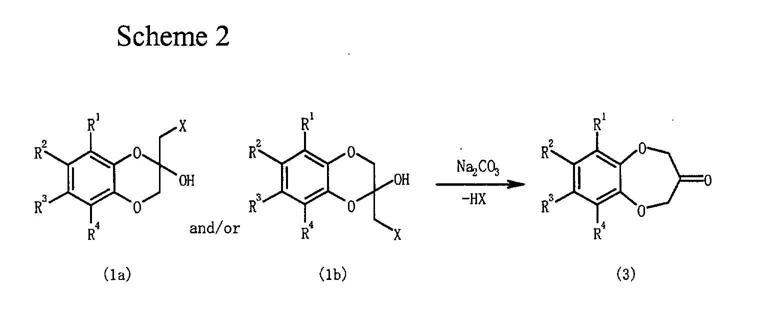

Examples of sodium carbonate include anhydrous sodium carbonate and sodium carbonate monohydrate. In order to improve the reaction rate, sodium carbonate is preferably made into fine powder particles. Sodium carbonate is preferably used in an amount ranging from about 0.2 to about 5.0 equivalents, preferably from about 0.5 to about 2.0 equivalents, relative to 2-hydroxy-2-halogenomethyl-1,4-benzodioxane derivatives of formula(e) (1a) and/or (1b).

Besides, a reaction accelerating agent can be used in the process of dehydrohalogenation reaction. Such a reaction accelerating agent includes, for instance, iodine salts e.g. sodium iodide and potassium iodide, phase-transfer catalysts, and the like. The reaction accelerating agent is preferably used in an amount ranging from about 0.01 to about 1.0 equivalent, preferably from about 0.05 to about 0.25 equivalent, relative to 2-hydroxy-2-halogenomethyl-1,4-benzodioxane derivatives (1a) and/or (1b).

Solvents for the reaction are not particularly limited, insofar as they do not affect the reaction. Examples of such solvents include water; alcohols such as methanol and ethanol; esters such as ethyl acetate; ketones such as acetone, methyl ethyl ketone, methyl isobutyl ketone and cyclohexanone; ethers such as diisopropyl ether, tetrahydrofuran and dioxane; aromatic hydrocarbons such as benzene, toluene and xylene; amides such as dimethylformamide; dimethyl sulfoxide; and the like. Each of the above solvents may be used alone, or as a mixture of at least two of them. Among the above solvents, ketones such as acetone, methyl ethyl ketone, methyl isobutyl ketone and cyclohexanone are preferably used.

The amount of solvent to be used is not particularly limited. However, for the purpose of industrial production, its weight ratio is chosen between about 3 : 1 and about 100 : 1, preferably between about 5 : 1 and about 50 : 1, relative to catechols of formula (5).

The reaction temperature is typically chosen between about 40°C and about 150°C, preferably between about 50°C and about 100°C.

The duration of reaction time is not particularly limited. However, for optimal industrial production, it is typically selected between about 1 and about 15 hours, preferably between about 3 and about 10 hours.

The reaction can be carried out in air, but preferably in an inert gas atmosphere. The inert gas includes, for example, nitrogen gas, argon gas and the like.

As mentioned supra, the end product of the inventive method is 3,4-dihydro(2H)-1,5-benzodioxepin-3-one of formula (3) below:

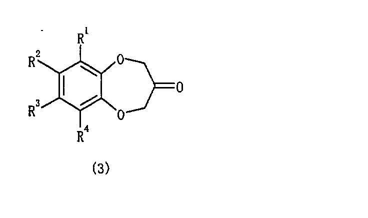

In the above formula, R¹, R², R³ and R⁴ respectively represent, independently, a hydrogen atom, an alkyl group or an alkenyl group. Further, when the alkyl groups, the alkenyl groups or the alkyl and alkenyl groups are placed adjacent, they may be linked together to form a new carbon ring. However, the total number of carbon atoms contained in R¹, R², R³ and R⁴ does not exceed 10. Preferred examples of R¹, R², R³ and R⁴ and newly formed carbon ring are as mentioned for the compound of formula (1a) or (1b).

Examples of the compound of formula (3) include:
6-methyl-3,4-dihydro(2H)-1,5-benzodioxepin-3-one;
7-methyl-3,4-dihydro(2H)-1,5-benzodioxepin-3-one;
6,8-dimethyl-3,4-dihydro(2H)-1,5-benzodioxepin-3-one;
7,8-dimethyl-3,4-dihydro(2H)-1,5-benzodioxepin-3-one;
7-ethyl-3,4-dihydro(2H)-1,5-benzodioxepin-3-one;
7-propyl-3,4-dihydro(2H)-1,5-benzodioxepin-3-one;
7-(2-propenyl)-3,4-dihydro(2H)-1,5-benzodioxepin-3-one;
7-butyl-3,4-dihydro(2H)-1,5-benzodioxepin-3-one;
7-(2-methylbutyl)-3,4-dihydro(2H)-1,5-benzodioxepin-3-one;
7-(3-methylbutyl)-3,4-dihydro(2H)-1,5-benzodioxepin-3-one;
7-pentyl-3,4-dihydro(2H)-1,5-benzodioxepin-3-one;
7-(2-methylpentyl)-3,4-dihydro(2H)-1,5-benzodioxepin-3-one;
7-(3-methylpentyl)-3,4-dihydro(2H)-1,5-benzodioxepin-3-one;
7-(4-methylpentyl)-3,4-dihydro(2H)-1,5-benzodioxepin-3-one;
7-hexyl-3,4-dihydro(2H)-1,5-benzodioxepin-3-one; etc., though not limited thereto.

Examples of preferred compounds of formula (3) include:
7-methyl-3,4-dihydro(2H)-1,5 -benzodioxepin-3-one;
7-ethyl-3,4-dihydro(2H)-1,5-benzodioxepin-3-one;
7-propyl-3,4-dihydro(2H)-1,5-benzodioxepin-3-one;
7-isopropyl-3,4-dihydro(2H)-1,5-benzodioxepin-3-one;
7-{2-propenyl)-3,4-dihydro(2H)-1,5-benzodioxepin-3-one;
7-butyl-3,4-dihydro(2H)-1,5-benzodioxepin-3-one;
7-(2-methylbutyl)-3,4-dihydro(2H)-1,5-benzodioxepin-3-one;
7-(3-methylbutyl)-3,4-dihydro(2H)-1,5-benzodioxepin-3-one;
7-pentyl-3,4-dihydro(2H)-1,5-benzodioxepin-3-one;
7-(2-methylpentyl)-3,4-dihydro(2H)-1,5-benzodioxepin-3-one;
7-(3-methylpentyl)-3,4-dihydro(2H)-1,5-benzodioxepin-3-one;
7-(4-methylpentyl)-3,4-dihydro(2H)-1,5-benzodioxepin-3-one; and 7-hexyl-3,4-dihydro(2H)-1,5-benzodioxepin-3-one.

One of the most preferred compounds comprises 7-methyl-3,4-dihydro(2H)-1,5-benzodioxepin-3-one. This compound is frequently used as one of a few fragrances comprising a marine note which invokes the image of the sea.

A third aspect of the invention relates to transforming the compounds of formulae (5) and (6) into 3,4-dihydro(2H)-1,5-benzodioxepin-3-ones (3) in one process step, as shown in Scheme 3 below:

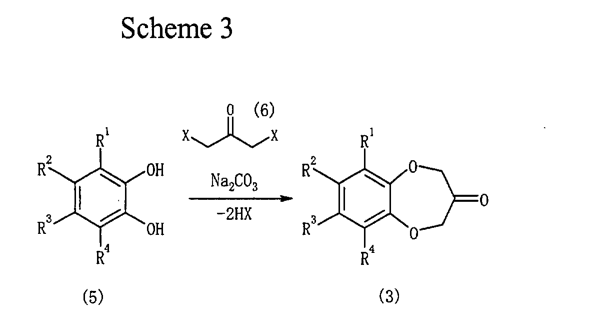

In other words, the cyclic compounds 3,4-dihydro(2H)-1,5-benzodioxepin-3-ones of formula (3) can efficiently be synthesized by one reaction step by reacting the compound of formula (5) with the compound of formula (6) in the presence of sodium carbonate. The sodium carbonate may be either anhydrous sodium carbonate or a hydrate, which is preferably prepared as fine powder particles.

In the above reaction, the amount of sodium carbonate ranges from about 0.7 to about 7 times, preferably from about 1.2 to about 6 times the molar ratio, relative to the compound of formula (5).

On the other hand, the compound of formula (5) is added to the compound of formula (6) in a weight ratio of about 1 : 1, preferably about 1 : 1.5.

Where appropriate, known reaction accelerating agents may be added to the reaction system. Such reaction accelerating agents include, for example, iodine salts e.g. sodium iodide or potassium iodide, phase-transfer catalysts and the like.

The solvents to be used in the reaction are not particularly limited, insofar as they do not affect the reaction. Examples of solvents include: water; alcohols such as methanol and ethanol; esters such as ethyl acetate; ketones such as acetone, methyl ethyl ketone, methyl isobutyl ketone and cyclohexanone; ethers such as diisopropyl ether, tetrahydrofuran and dioxane; aromatic hydrocarbons such as benzene, toluene and xylene; amides such as dimethylformamide; and dimethyl sulfoxide; and the like. Each of these solvents may be used alone or as a mixture of at least two of them. Among the above solvents, ketones such as acetone, methyl ethyl ketone, methyl isobutyl ketone and cyclohexanone are preferably used.

The amount of solvent to be used is not particularly limited. However, the more diluted a reaction solution is, the more easily the formation of chain polymers, due to intermolecular reaction, is prevented. Conversely, a reaction solution, when denser, gives an economical edge from an industrial point of view. Accordingly, the weight ratio of a solvent to compound (5) is appropriately selected generally between about 3 : 1 and about 200 : 1, preferably between about 5 : 1 and about 50 : 1, relative to the solute.

The reaction temperature is typically set between about 30°C and about 160°C, preferably between about 40°C and about 100°C.

The length of reaction time is typically selected between about 1 and about 20 hours.

The reaction can be carried out in air, but preferably in an inert gas atmosphere. The inert gas includes, for example, nitrogen gas, argon gas and the like.

The catechols and 1,3-dihaloacetones used are those represented by the formula (5) and the formula (6), respectively, and described supra.

### [Examples]

Now, the invention is described with reference to Examples and Comparative Examples. It is, however, to be understood that the invention is not limited to the particulars disclosed herebelow.

The instruments used and the measuring conditions applied are as follows.
(1) Infrared absorption spectrum (IR):
   Instrument: Nicolet FT-IR AVATAR 360
   Neat, NaCl film
(2) Nuclear magnetic resonance spectrum (NMR):
   Instrument 1): AMX-500 (500 MHz) (manufactured by Broker)
   Internal standard: tetramethylsilane;
   Solvent: CDCl₃.
(3) Mass spectrum (MS):
   Instrument: TRIO-1000 (manufactured by VG)
   Column: BC WAX (0.25 mm × 60 m)
   Carrier gas: helium
   Measuring temperature: 70°C to 220°C (heated at a rate of 4°C/min)
(4) Gas chromatography (GC):
   Instrument: HP-5890A (manufactured by Hewlett Packard)
   Column: HP-20M (0.25 mm x 25 m) (manufactured by Hewlett Packard)
   Carrier gas: helium
   Measuring temperature: 55°C to 215°C (heated at a rate of 4°C/min)
   Detector: flame ionization detector

### [Example 1] 2-hydroxy-2-chloromethyl-1,4-benzodioxane

20.57 g of Na₂CO₃ (0.194 mol) and 3.4 g of KI (0.02 mol) were suspended in methyl ethyl ketone (510 ml), and vigorously stirred. To this vigorously stirring suspension, 20.32 g of 1,3-dichloroacetone (0.160 mol) and 15.0 g of catechol (0.136 mol), dissolved in 80 ml of methyl ethyl ketone, were added dropwise over a period of 1 hour, under reflux at 80°C in nitrogen atmosphere.

After the addition, the reaction mixture was further refluxed for 10 hours and cooled down. The mixture was then filtered to remove inorganic salt precipitates, and the filtrate was distilled under reduced pressure to give 35 g of brownish black oil. The oil was subjected to column chromatography on silica gel using CH₂Cl₂ as solvent, to give 17 g of crude product of 2-hydroxy-2-chloromethyl-1, 4-benzodioxane (purity: 80% by GC). The crude product was further purified by column chromatography on silica gel using CH₂Cl₂ as solvent, to obtain a pure product of 2-hydroxy-2-chloromethyl-1,4-benzodioxane. The spectrum data of the pure product are as follows.
IR (neat, NaCl, cm⁻¹): 3372, 2967, 1598, 1495, 1464, 1262, 1141, 1107, 1083, 1045, 947, 842, 752
MS (m/e, 20 eV): 52, 63, 65, 77, 81, 95, 109, 110, 121, 123, 161, 165, 183, 200(M⁺), 202 (M⁺+2)
NMR (CDCl₃, 500 MHz, ppm): 3.71 (1H,d,J=11.9Hz), 3.76 (1H,d,J=11.9Hz), 4.08 (1H,d,J=11.2Hz), 4.16 (1H,d,J=11.2Hz), 6.89-6.96 (4H,m)
¹³C-NMR (CDCl₃, 500 MHz, ppm): 46.19, 67.23, 92.86, 117.17, 117.66, 122.3, 122.7, 140.97, 141,87

### [Example 2] Synthesis of 3 4-Dihydro(2H)-1,5-benzodioxepin-3-one from 2-hydroxy-2-chloromethyl-1,4-benzodioxane

3.0 g of Na₂CO₃ (0.0283 mol), 0.48 g of KI (0.003 mol) and 4.0 g (0.016 mol; Purity:80%) of 2-hydroxy-2-chloromethyl-1,4-benzodioxane prepared in Example 1 were suspended in acetone (87 ml). The obtained suspension was refluxed for 7 hours under vigorous stirring in nitrogen atmosphere. The reaction mixture was then cooled down to room temperature. The mixture was filtered to remove inorganic salt precipitates, and the filtrate was distilled under reduced pressure, to give 3.72 g of brownish red oil as a crude product.

Vacuum distillation of the crude product, performed under the conditions e.g. bp: 74°C/0.15 torr (20Pa), gave 1.9 g of 3,4-dihydro(2H)-1,5-benzodioxepin-3-one.
m.p. 36°C (after recryslallization from diisopropyl ether)
MS (20 eV, m/e): 52, 63, 80, 94, 108, 121, 136, 164 (M⁺)
¹H-NMR (CDCl₃, ppm): 4.71 (4H,s), 6.96-7.02 (4H,m)

### [Example 3] Synthesis of a mixture of 2-hydroxy-2-chloromethyl-6-methyl-1,4-benzodioxane and 2-hydroxy-2-chloromethyl-7-methyl-1,4-benzodioxane

6.00 g of Na₂CO₃ (0.057 mol) and 1.00 g of KI (0.006 mol) were suspended in acetone (150 ml), and vigorously stirred. To this stirring suspension, 5.95 g of 1,3-dichloroacetone (0.047 mol) and 5.00 g of 4-methylcatechol (0.040 mol), dissolved in 25 ml of acetone, were added dropwise over a period of 3 hours, under reflux at 56°C in nitrogen atmosphere.

The reaction mixture was refluxed for 1 hour and cooled down. The mixture was then filtered to remove inorganic salt precipitates, and the filtrate was distilled under reduced pressure to give 8.92 g of dark-colored oil.

The dark-colored oil was subjected to chromatography on silica gel and eluted with dichloromethane, to give 7.69 g (0.036 mol) of roughly equimolecular mixture of 2-hydroxy-2-chloromethyl-6-methyl-1,4-benzodioxane and 2-hydroxy-2-chloromethyl-7-methyl-1,4-benzodioxane (Yield: 88.95%).

According to the spectral data, the above mixture comprises 6- and 7-methyl derivatives at a molar ratio of about one to one, the derivatives having intramolecular hemiketal structures.
IR (neat, cm⁻¹): 3436, 1600, 1509, 1425, 1275, 1200, 1075, 1050, 950, and 810.
¹H-NMR (CDCl₃, ppm): 2.26 and 2.26* (3H,s), 3.70 and 3.71 (1H,d,J=11.9 Hz), 3.76 and 3.76* (1H,d,J=11.9 Hz), 4.06 and 4.06* (1H,d,J=11.0 Hz) 4.13 and 4.14 (1H,d,J=11.0 Hz), 6.70 and 6.72 (1H,dd,J=8.2 and 1.8 Hz) 6.74 and 6.76 (1H,d,J=1.8 Hz), 6.81 and 6.83 (1H,d,J=8.2 Hz), (*: Difference of chemical shift of these two peaks is smaller than 0.01 ppm).
¹³C-NMR (CDCl₃, ppm): 20.6 and 20.7, 46.28 and 46.3, 67.4, 92.7 and 92.9, 116.8 and 117.3, 117.5 and 118.0, 122.8 and 123.2, 132.0 and 132.5, 138.7 and 139.6, 140.6 and 141.5/
MS m/e (%): 66(60), 77(54), 91(42), 95(44), 109(79), 123(100), 124(100), 135(44), 178(3), 214(100,M+), 216(53,M+2).

### [Example 4] 7-Methyl-3,4-dihydro(2H)-1,5-benzodioxepin-3-one

5.12 g of Na₂CO₃ (0.048 mol) and 0.60 g of potassium iodide (0.004 mol) were added to 60 ml of acetone. To this mixture, 3.68 g of 2-hydroxy-2-chloromethyl-6-methyl-1,4-benzodioxane and 2-hydroxy-2-chloromethyl-7-methyl-1,4-benzodioxane (0.017 mol), dissolved in 10 ml of acetone, were added dropwise over a period of 4 hours, under reflux at 56°C with stirring, in nitrogen atmosphere.

The reaction mixture was further refluxed for 8 hours, and cooled down. The mixture was filtered to remove inorganic salt precipitates, and the filtrate was distilled under reduced pressure, to give 3.69 g of dark-colored oil.

The dark-colored oil was subjected to chromatography on silica gel and eluted with dichloromethane, to give 2.58 g (0.014 mol) of 7-methyl-3,4-dihydro(2H)-1,5-benzodioxepin-3-one (yield: 84.45%).

### [Example 5] 7-Methyl-3,4-dihydro(2H)-1,5-benzodioxepin-3-one

5.12 g of Na₂CO₃ (0.048 mol) and 0.60 g of potassium iodide (0.004 mol) were suspended in 60 ml of acetone. To this suspension, 3.68 g of 1,3-dichloroacetone (0.029 mol) and 3.00 g of 4-methylcatechol (0.024 mol), dissolved in 10 ml of acetone, were added dropwise over a period of 4 hours, under reflux at 56°C with stirring, in nitrogen atmosphere. The reaction mixture was refluxed for 8 hours, and cooled down. The mixture was then filtered to remove inorganic salt precipitates, and the filtrate was distilled under reduced pressure, to give 3.69 g of a dark-colored oil.

The dark-colored oil was applied to chromatography on silica gel and eluted with dichloromethane, to give 2.58 g (0.014 mol) of 7-methyl-3,4-dihydro(2H)-1,5-benzodioxepin-3-one (yield: 59.91%).

### [Comparative Examples 1 through 5]

Compounds for Comparative Examples were synthesized in the same manner as in Example 5, except that sodium carbonate in Example 5 is replaced by the salts indicated in Table 1. The amounts of salts used and the obtained product yields are also shown in Table 1.

**Table 1**

| | Salts | Amount | Yield |
|---|---|---|---|
| Comparative Example 1 | lithium carbonate | 3.57 g (0.048 mol) | 0% |
| Comparative Example 2 | potassium carbonate | 6.68 g (0.048 mol) | 10% |
| Comparative Example 3 | cesium carbonate | 18.64 g (0.048 mol) | 0% |
| Comparative Example 4 | sodium hydroxide | 1.93 g (0.048 mol) | 0% |
| Comparative Example 5 | sodium bicarbonate | 4.06 g (0.048 mol) | 5% |

### [Example 6] 7-Methyl-3,4-dihydro(2H)-1,5-benzodioxepin-3-one

Anhydrous Na₂CO₃ (69.58 g; 0.656 mol, 200 mesh), potassium iodide (8.17 g; 0.049 mol) and 4-methylcatechol (40.74 g; 0.328 mol) were suspended in methyl ethyl ketone (1804 ml). To this suspension, 1,3-dichloroacetone (50.0 g; 0.394 mol), dissolved in methyl ethyl ketone (164 ml), was added dropwise over a period of 150 minutes, under reflux with stirring in nitrogen atmosphere.

The reaction mixture was refluxed for 100 minutes, cooled down to 10°C and filtered through a pad of filter aid. The filtrate was distilled at 60°C under a pressure of 100 torr (13332 Pa). To the resultant residue were added, successively, water (100 ml), toluene (200 g), a 20 % NaCl solution (100 ml) and acetic acid (5.0 g). The mixture was stirred, and the resulting organic layer was separated and washed with a 20 % NaCl solution (100 ml).

The recovered organic layer was distilled under reduced pressure (30 mmHg), to give a residue (94.0 g). The residue was then distilled to give a crude product (79.23 g). The crude product was further distilled at 77.5-84°C/0.3-0.15 torr (40-20 Pa), to yield 7-methyl-3,4-dihydro(2H)-1,5-benzodioxepin-3-one (49.48 g: purity 90.11 %, yield: 76.24 %).

### [Example 7] 7-Methyl-3,4-dihydro(2H)-1,5benzodioxepin-3-one

2.12 g of Na₂CO₃ (0.02 mol) and 0.249 g of potassium iodide (0.0015 mol) were mixed in cyclohexanone (80 ml). To this mixture, 1.52 g of 1,3-dichloroacetone (0.012 mol) and 1.24 g of 4-methylcatechol (0.010 mol), dissolved in 10 ml of cyclohexanone, were added dropwise over a period of 95 minutes, under reflux with stirring in nitrogen atmosphere. The reaction mixture was refluxed for 4 hours, and cooled down to room temperature. The mixture was filtered to remove inorganic salt precipitates.

The progress of the reaction was checked by gas chromatography using an internal standard. 7-Methyl-3,4-dihydro(2H)-1,5-benzodioxepin-3-one was obtained with a yield of 75.25 %.

### [Example 8] 7-Methyl-3,4-dihydro(2H)-1,5-benzodioxepin-3-one

5.12 g of Na₂CO₃ (0.048 mol) and 0.60 g of potassium iodide (0.004 mol) were mixed in acetone (97.5 ml) in a flask. To this mixture, 6.26 g of 1,3-dibromoacetone (0.029 mol) and 3.00 g of 4-methylcatechol (0.024 mol), dissolved in 10 ml of acetone, were added dropwise over a period of 4 hours, under reflux with stirring in nitrogen atmosphere. To this end, the flask supra was filled with nitrogen, and the reaction mixture was stirred and refluxed at 56°C.

The reaction mixture was further refluxed for 8 hours, and cooled down. The mixture was then filtered to remove inorganic salt precipitates, and the filtrate was distilled under reduced pressure to give a dark-colored oil.

The dark-colored oil was applied to chromatography on silica gel and eluted with dichloromethane, to give 2.84 g of 7-methyl-3,4-dihydro(2H)-1,5-benzodioxepin-3-one (yield: 69.95 %).

### [Example 9] 3,4-Dihydro(2H)-1,5-benzodioxepin-3-one

5.12 g of Na₂CO₃ (0.048 mol) and 0.60 g of potassium iodide (0.004 mol) were mixed in acetone (60 ml). To this mixture, 3.68 g of 1,3-dichloroacetone (0.029 mol) and 2.64 g of catechol (0.024 mol), dissolved in acetone (10 ml), were added dropwise over a period of 4 hours, under reflux with stirring in nitrogen atmosphere.

The reaction mixture was refluxed for 8 hours, and cooled down. The mixture was then filtered to remove inorganic salt precipitates, and the filtrate was distilled under reduced pressure to give a dark-colored oil.

The dark-colored oil was applied to chromatography on silica gel and eluted with dichloromethane, to give 2.38 g (0.014 mol) of 3,4-dihydro(2H)-1,5-benzodioxepin-3-one (yield: 60.47 %).

### [Examples 10 through 12]

Compounds of Examples 10 to 12 were obtained in the same manner as in Example 9, except that catechol of these Examples is replaced by catechol derivatives shown in Table 2. The amounts of derivatives used and the obtained product yields are shown in Table 2.

**Table 2**

| | Catechol | Amount | Yield |
|---|---|---|---|
| Example 10 | 4-propylcatechol | 3.64 g (0.024 mol) | 58.40% |
| Example 11 | 4-allylcatechol | 3.60 g (0.024 mol) | 61.30% |
| Example 12 | 4-(3-methylbutyl)-catechol | 4.32 g (0.024 mol) | 56.80% |

As has been described in the foregoing, 3,4-dihydro(2H)-1,5-benzodioxepin-3-ones, produced by the method of the invention, have a fragrance comprising a marine note. The products can be produced by a single process step by either:
1) reacting catechols with 1,3-dihaloacetones in the presence of sodium carbonate under mild reaction conditions, thereby obtaining novel cyclic compounds represented by the formula (1a) and/or formula (1b), and further reacting the novel cyclic compounds with sodium carbonate; or
2) reacting catechols with 1,3-dihaloacetones in the presence of sodium carbonate, up to the end of the reaction without isolating the novel cyclic compounds of formula (1a) and/or formula (1b).

3,4-Dihydro(2H)-1,5-benzodioxepin-3-ones can thus be produced efficiently and economically by the inventive method which has marked advantage, compared to the conventional methods which require several reaction steps.

## Claims

1. A method for producing 3,4-dihydro(2H)-1,5-benzodioxepin-3-one represented by the formula (3): 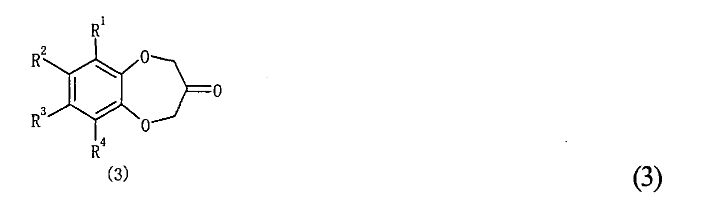 wherein R¹, R², R³ and R⁴ respectively represent, independently, a hydrogen atom, an alkyl group or an alkenyl group, with the proviso that R¹, R², R³ and R⁴ as a whole do not comprise more than 10 carbon atoms and that, when the two adjacent groups respectively comprise an alkyl group or an alkenyl group, they may be combined together to form a carbon ring;
**characterised by** comprising the step of reacting catechols represented by the formula (5): 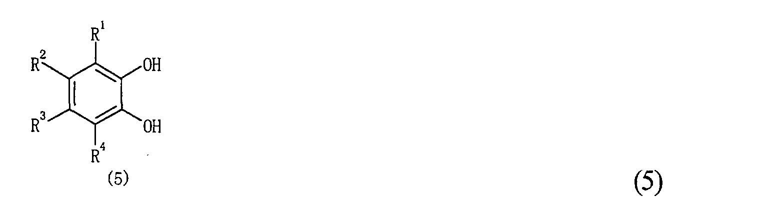 wherein R¹, R², R³ and R⁴ are as defined above;
with 1,3-dihaloacetones represented by the formula (6): 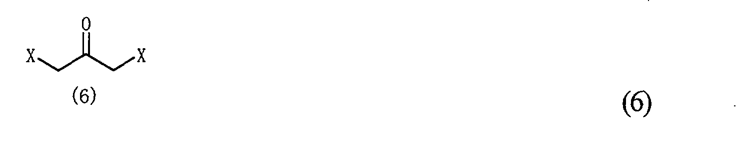 wherein the two letters X represent, independently, a halogen atom; in the presence of sodium carbonate, through the removal of hydrogen halide.

2. A method for producing 2-hydroxy-2-halogenomethyl-1,4-benzodioxane derivatives represented by the formula (1a): 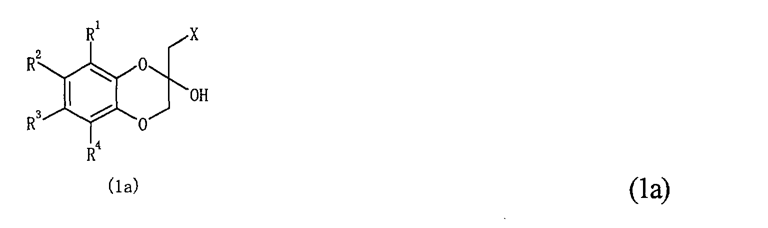 wherein X represents a halogen atom, and R¹, R², R³ and R⁴ respectively represent, independently, a hydrogen atom, an alkyl group or an alkenyl group, with the proviso that R¹, R², R³ and R⁴ as a whole do not comprise more than 10 carbon atoms and that, when the two adjacent groups respectively comprise an alkyl group or an alkenyl group, they may be combined together to form a carbon ring;
and/or 2-hydroxy-2-halogenomethyl-1,4-benzodioxane derivatives represented by the formula (1b): 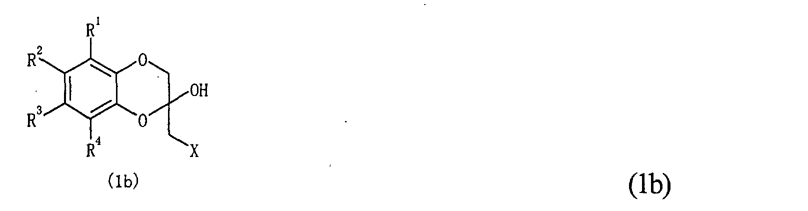 wherein R¹, R², R³, R⁴ and X are as defined above;
**characterised by** comprising the step of reacting catechols represented by the formula (5): 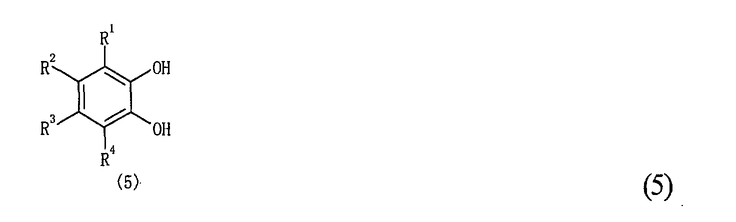 wherein R¹, R², R³ and R⁴ are as defined above;
with 1,3-dihaloacetones represented by the formula (6): 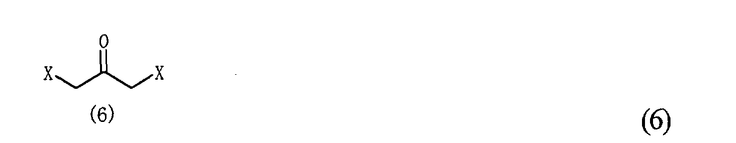 wherein the two letters X represent, independently, a halogen atom;
in the presence of sodium carbonate, through the removal of hydrogen halide.

3. The method according to claim 1 or 2, wherein said reacting step comprises reacting said catechols with 1,3-dihaloacetones in which said halogen atom comprises a chlorine atom or a bromine atom.

4. The method according to any one of claims 1 to 3, wherein said reacting step comprises reacting, with said 1,3-dihaloacetones, catechols in which R¹ and R⁴ are hydrogen atoms and either R² or R³ is a hydrogen atom and the other is an alkyl group or alkenyl group.

5. A method for producing 3,4-dihydro(2H)-1,5-benzodioxepin-3-one represented by the formula (3): 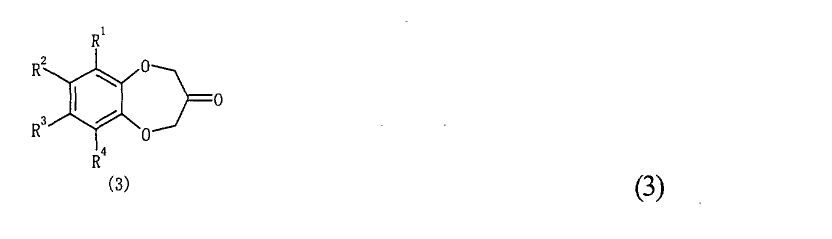 wherein R¹, R², R³ and R⁴ respectively represent, independently, a hydrogen atom, an alkyl group or an alkenyl group, with the proviso that R¹, R², R³ and R⁴ as a whole do not comprise more than 10 carbon atoms and that, when the two adjacent groups respectively comprise an alkyl group or an alkenyl group, they may be combined together to form a carbon ring;
**characterised by** comprising the step of reacting at least one compound represented by the formula (1a): 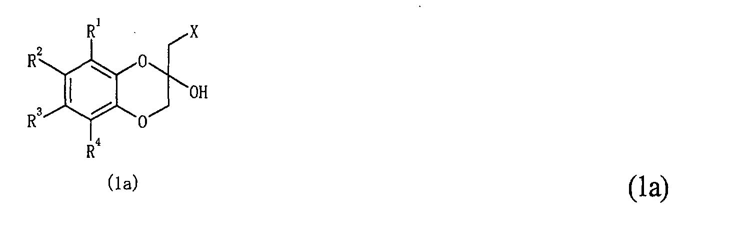 wherein X represents a halogen atom; and R¹, R², R³ and R⁴ are as defined above;
or at least one compound represented by the formula (1b): 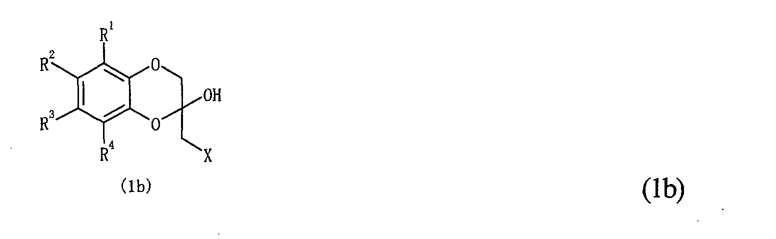 wherein X, R¹, R², R³ and R⁴ are as defined above;
or a mixture thereof;
with sodium carbonate, through the removal of hydrogen halide.

6. The method according to claim 5, wherein said reacting step comprises reacting, with said sodium carbonate, at least one compound of formula (1a) or (1b) in which said halogen atom comprises a chlorine atom or a bromine atom.

7. The method according to claim 5 or 6, wherein said reacting step comprises reacting, with said sodium carbonate, at least one compound of formula (1a) or (1b) in which R¹ and R⁴ are hydrogen atoms and either R² or R³ is a hydrogen atom and the other is an alkyl group or alkenyl group.

8. A 2-hydroxy-2-halogenomethyl-1,4-benzodioxane derivative represented by the formula (1a): 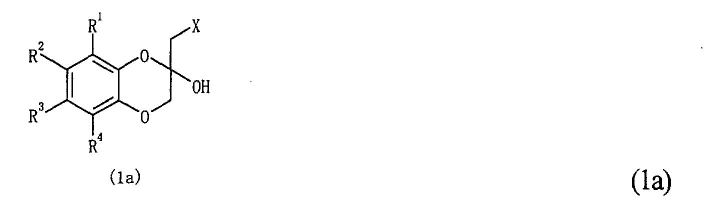 wherein X represents a halogen atom; R¹, R², R³ and R⁴ respectively represent, independently, a hydrogen atom, an alkyl group or an alkenyl group, with the proviso that R¹, R², R³ and R⁴ as a whole do not comprise more than 10 carbon atoms and that, when the two adjacent groups respectively comprise an alkyl group or an alkenyl group, they may be combined together to form a carbon ring;
or represented by the formula (1b): 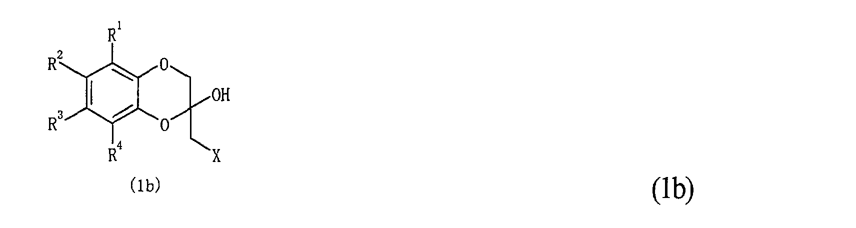 wherein X, R¹, R², R³ and R⁴ are as defined above.

9. The 2-hydroxy-2-halogenomethyl-1,4-benzodioxane derivative according to claim 8, wherein said halogen atom comprises a chlorine atom or a bromine atom.

10. The 2-hydroxy-2-halogenomethyl-1,4-benzodioxane derivative according to claim 8 or 9, wherein R¹ and R⁴ are hydrogen atoms and either one of R² and R³ is hydrogen atom and the other is an alkyl group or alkenyl group.
